# EUROPEAN PATENT SPECIFICATION

(11) **EP 2 342 186 B1**
(45) Date of publication and mention of the grant of the patent: **17.09.2014**
(21) Application number: 10791750.2
(22) Date of filing: 18.06.2010
(51) Int. Cl.: C07D 239/47, C07D 239/46, A61K 31/505

(54) **PROCESS FOR SYNTHESIS OF ETRAVIRINE**
VERFAHREN ZUR SYNTHESE VON ETRAVIRIN
PROCÉDÉ POUR LA SYNTHÈSE D'ETRAVIRINE

(30) Priority: 22.06.2009 IN MU14792009
(43) Date of publication of application: 13.07.2011
(73) Proprietor: Emcure Pharmaceuticals Limited, Pune, Maharashtra 411026 (IN)
(72) Inventor: GURJAR, Mukund Keshav, Bhosari Pune 411 026 (IN); MAIKAP, Golakchandra Sudarshan, Bhosari Pune 411 026 (IN); JOSHI, Shashikant Gangaram, Bhosari Pune 411 026 (IN); PARDESHI, Devising Rameshsing, Bhosari Pune 411 026 (IN); KAMBLE, Mangesh Gorakhanath, Bhosari Pune 411 026 (IN); MEHTA, Samit Satish, Bhosari Pune 411 026 (IN)
(74) Representative: Engelhard, Markus
(86) International application number: PCT/IN2010/000422
(87) International publication number: WO 2010/150279

(56) References cited:
- WO-A1-2006/087387
- WO-A2-2008/016522
- WO-A2-2010/131118
- DE SPIEGELEER B ET AL: "Synthesis and HPLC purification of [77Br]TMC123-R165335 (etravirine), a new anti-HIV drug of the DAPY-NNTRI class", JOURNAL OF LABELLED COMPOUNDS AND RADIOPHARMACEUTICALS, JOHN WILEY, CHICHESTER, GB, vol. 49, 1 January 2006 (2006-01-01), pages 683-686, XP002510173, ISSN: 0362-4803, DOI: 10.1002/JLCR.1085
- BART DE SPIEGELEER ET AL: 'Synthesis and HPLC-purification of [77Br]TMC125-R165335 (etravirine), a new anti-HIV drug of the DAPY-NNRTI class' JOURNAL OF LABELLED COMPOUNDS AND RADIOPHARMACEUTICALS vol. 49, no. 8, 2006, pages 683 - 686, XP002510173
- DONALD W. LUDOVICI ET AL: 'Evolution of Anti-HIV Drug Candidates. Part 3: Diarylpyrimidine(DAPY) Analogues' BIOORGANIC AND MEDICINAL CHEMISTRY LETTERS vol. 11, 2001, pages 2235 - 2239, XP002329859

## Description

### FIELD OF INVENTION

The present invention relates to a method for synthesis of non-nucleoside reverse transcriptase inhibitor. In particular, the present invention relates to a method for synthesis of diarylpyrimidines non-nucleoside reverse transcriptase inhibitor such as etravirine.

### BACKGROUND OF THE INVENTION

Non-nucleoside reverse transcriptase inhibitors (NNRTIs) are a key component of highly active antiretroviral therapy (HAART) because of their ability to target an allosteric binding pocket on the reverse transcriptase enzyme giving rise to a broad spectrum of activity against HIV RT mutations. HAART has been the standard of care for Human Immunodeficiency Virus (HIV) infection since 1996 and has resulted in substantial increases in survival. Diarylpyrimidine compounds represent second generation NNRTIs and are useful for treatment of HIV infected patients with NNRTI-resistant viruses. Etravirine (I), formerly TMC 125 and chemically known as 4-[[6-amino-5-bromo-2-[(4-cyanophenyl)amino]-4-pyrimidinyl]oxy]-3,5-dimethylbenzonitrile, is an NNRTI approved in 2008 for use in combination with other antiretroviral agents in treatment-experienced adult patients with multidrug-resistant HIV infections. Etravirine is marketed worldwide as an oral tablet and was first disclosed by De Corte et al in US 7,037,917.

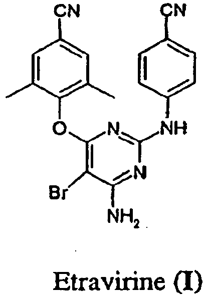

De Corte et al in US 7,037,917 provides a method for manufacturing of diarylpyrimidine compounds wherein a compound of formula (II) is heated with ammonia in presence of a inert solvent such as 1,4-dioxane in a pressure vessel at 150°C for 4 days.

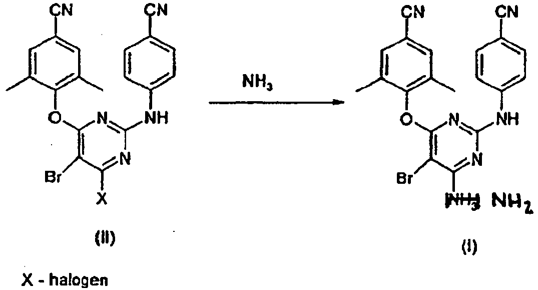

Davies et al in Drugs of the Future 2005, 30(5): 462-468 discloses that the intermediate compound (II) can be prepared in two different routes. The first route discloses that 5-bromo-2,4,6-trichloropyrimidine is reacted with 4-aminobenzonitrile by means of diisopropylethylamine in refluxing dioxane giving a diarylamine which is then reacted with 4-hydroxy-3,5-dimethylbenzonitrile to give intermediate of formula (II) (Scheme - 1).

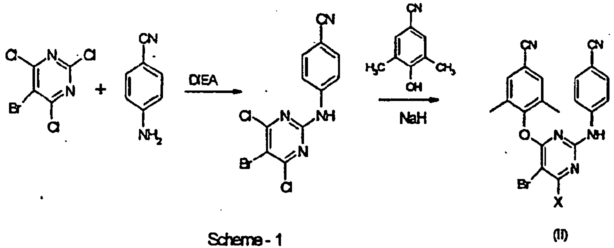

The second method for synthesis of compound of formula (II) discloses that 4-guanidinobenzonitrile is cyclized with diethylmalonate by means of sodium ethoxide to give 4-(4,6-dihydroxypyrimidine-2-yl-amino)-benzonitrile, which upon treatment with POCl₃ yields the corresponding dichloro derivative. Further bromination with bromine and sodium bicarbonate in aqueous methanol affords 4-(5-bromo-4,6-dichloropyrimidin-2-ylamine)-benzonitrile, which on condensation with the sodium salt of cyano-2,6-dimethylphenolate in presence of N-methylpyrrolidone and dioxane gives intermediate of formula (IIa) (Scheme - 2). 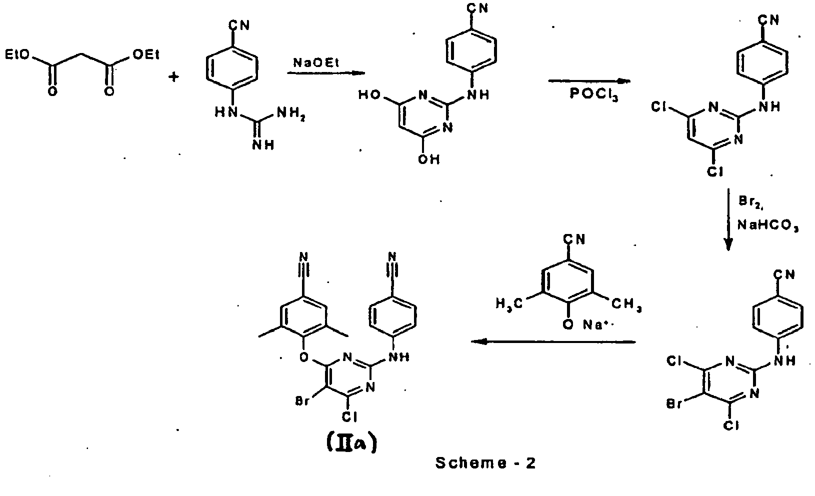 However, the abovementioned procedure for synthesis of diarylpyrimidine NNRTIs suffers from the disadvantage that the conversion of compound of formula II to the final compound is very slow. The reaction of compound of formula (II) with ammonia, even in refluxing dioxane requires four days for completion and the yields obtained are not very satisfactory.

Recently, De Kock et al, in US 2008/0194602 has reported that diarylpyrimidine oxide derivatives possesses HIV replication inhibiting properties. The diarylpyrimidine oxide derivatives are prepared from corresponding diarylpyrimidine derivatives of formula III by N-oxidation of the tertiary nitrogen of pyrimidine ring, wherein RI is fluorine, chlorine, bromine or iodine and R2 and R3 each independently are a C₁-C₆ alkyl.

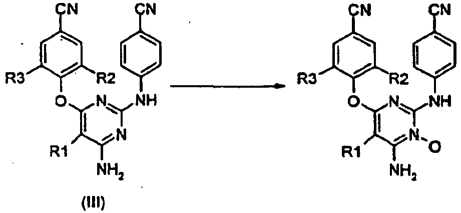

There are various methods reported in US 2008/0194602 for synthesis of diarylpyrimidine derivatives of formula III, as summarized in Scheme - 3.

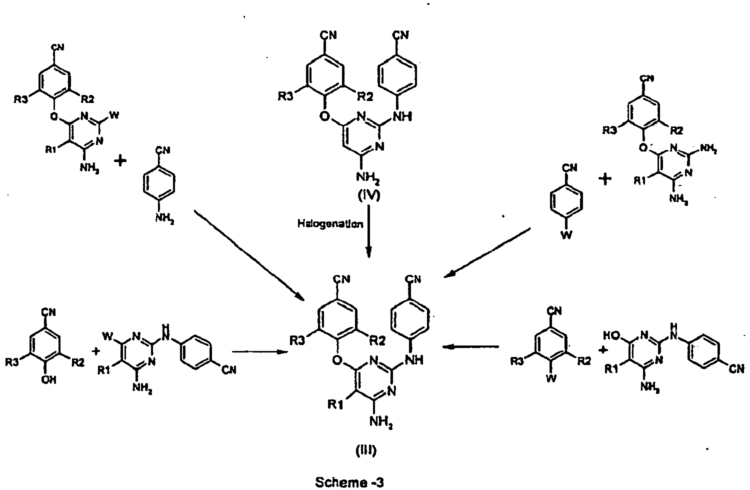

One of the most preferred processes reported in US 2008/0194602 for synthesis of diarylpyrimidine derivatives of formula (III), is by halogenating a compound of formula (IV): Further; the-compound of formula (IV) is reported to be prepared from 4-aminobenzonitrile and cyanamide. This reaction is conducted in water in the presence of a strong acid, to yield 4-cyanophenyl guanidine, which is then reacted with an alkyl malonic ester, in the presence of a strong base and at increased temperature. The obtained 4,6-dihydroxypyrimidine is then treated with a halogenating agent. The pyrimidine derivative is then reacted with a 4-substituted benzonitrile and then further with ammonia to yield the intermediates (IV).

Although, the process disclosed in US 2008/0194602 relates to synthesis of N-oxide derivatives and not particularly etravirine, the same method could be used for synthesis of etravirine. However, the said process again suffers from the limitation that it utilizes cyanamide, which is a highly toxic compound.

J. Label Compd Radiopharm 2006; 49; 683-686 teaches the preparation of etravirin from its desbromo derivative (compound of formula IVa)

Therefore, there exists a need to develop an alternative, cost effective and safe method of synthesis of diarylpyrimidine NNRTIs.

### OBJECTS OF THE INVENTION

An object of the present invention is to provide a simple, cost effective, and efficient process for synthesis of diarylpyrimidine NNRTIs.

Another object of the present invention is to provide a simple, cost effective, and efficient process for synthesis of etravirine.

Yet another object of the present invention is to provide a method for synthesis of etravirine using a compound of formula (V) i.e. 4-[(2,6-dichloro)-4-pyrimidinyloxy]-3,5-dimethylbenzonitrile.

A still further object of the present invention is to provide a simple method for synthesis of compound of formula (VI), 4-[[6-chloro-2-[(4-cyanophenyl)amino]-4-pyrimidinyl]oxy]-3,5-dimethylbenzonitrile from a compound of formula (V) i.e. 4-[(2,6-dichloro)-4-pyrimidinyloxy]-3,5-dimethylbenzonitrile.

### SUMMARY OF THE INVENTION

The scope of the present invention is defined in the appended claims.

In their endeavor to prepare a simple, cost effective method for synthesis of etravirine, the present inventors have found that diarylpyrimidine NNRTIs could be prepared by using inexpensive starting material such as 2,4,6-trichloropyrimidine and hydroxybenzonitrile derivative. The obtained pyrimidine derivative is then treated with suitable aminobenzonitrile derivatives to get intermediate diarylpyrimidine compounds, which could be readily converted into diarylpyrimidine NNRTIs having anti HIV activity.

The present inventors have developed a simple and commercially viable process for synthesis of etravirine. A typical process for synthesis of etravirine comprises of steps of:
1. Condensing 2,4,6-trichlorpyrimidine with 3,5-dimethyl-4-hydroxybenzonitrile to obtain compound of formula (V);
2. Converting the compound of formula (V) to a compound of formula (VI) by condensation with 4-aminobenzonitrile;
3. Ammonlysis of compound of formula (VI) to get a compound of formula (IVa); and
4. Halogenation of compound of formula (IVa) to get Etravirine.

The present invention also provides a novel, simple and efficient method for conversion of compound of formula (V) to a compound of formula (VI). Typically, the said process comprises of condensing a compound of formula (V) with 4-aminobenzonitrile in a suitable solvent to get a compound of formula (VI).

Thus, the present invention provides a simple, economical, efficient process, suitable for large scale production, process for synthesis of diarylpyrimidine NNRTIs.

In another aspect, the present invention provides a commercially viable process for synthesis of etravirine.

In still further aspect, the present invention provides a simple process for conversion of compound of formula (V) to a compound of formula (VI).

### DETAILED DESCRIPTION OF THE INVENTION

The present invention relates to a simple, economical and commercially viable process for the preparation of diarylpyrimidine NNRTIs. In particular, the present invention relates to a novel method for synthesis of etravirine. Typically, the instant invention provides a method for synthesis of etravirine using a compound of formula (V) i.e. 4-[(2,6-dichloro)-4-pyrimidinyloxy]-3,5-dimethylbenzonitrile and 4-aminobenzonitrile as shown in Scheme - 4. 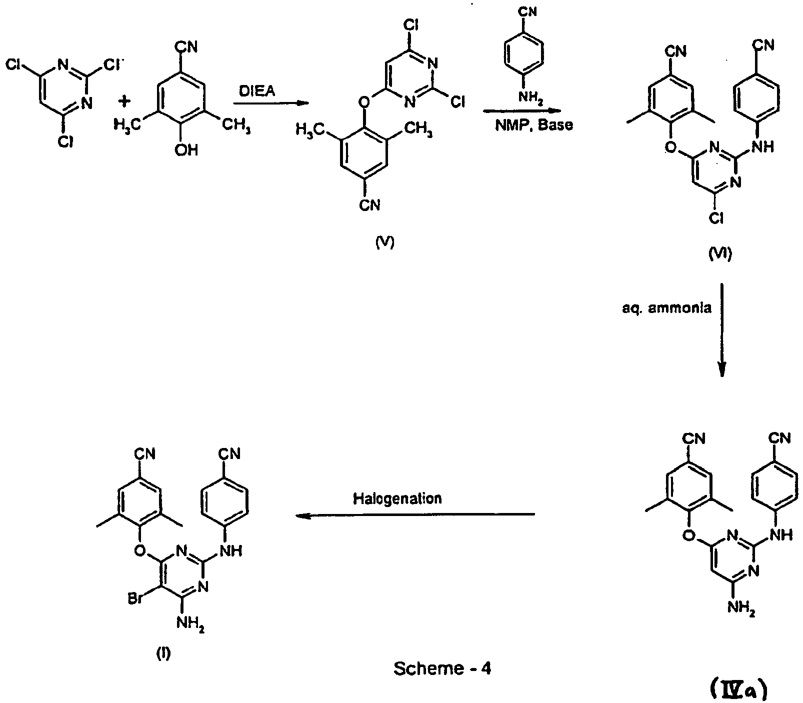 In the above procedure, 2,4,6 - trichloropyrimidine is reacted with 3,5-dimethyl-4-hydroxybenzonitrile, to obtain a compound of formula (V), in an inert solvent such as ethanol, N-methyl-2-pyrrolidone, N,N-dimethylformamide, 1,4-dioxane, tetrahydrofuran, dimethylsulfoxide, tetraline, sulfolane, acetonitrile and the like. The reaction is preferably carried out at refluxing temperatures and optionally, in presence of base. Preferably, 1,4-dioxane is used as solvent and N,N-diisopropylethylamine as base.

The obtained compound of formula (V) is then condensed with 4-aminobenzonitrile to give a compound of formula (VI). The condensation of compound of formula (V) with 4-aminobenzonitrle is the most critical step of the present invention. The present inventors have found that acidic conditions are not typically favorable for the instant reaction. When the said condensation reaction is carried out in presence of acid catalyst such as 1N HCl and an inert solvent such as dimethylformamide or N-methyl pyrrolidone, the reaction does not proceed smoothly as desired yielding the desired products. Even in presence of inorganic bases such as sodium hydroxide, potassium hydroxide, sodium carbonate, potassium carbonate reactions fails. Presence of organic bases such as diethylamine, pyridine, dibutyl urea also fails to initiate the condensation reaction.

However, present inventors have surprisingly found that the condensation reaction of compound of formula (V) with 4-aminobenzonitrile to give a compound of formula (VI) could be carried out in presence of alkoxides such as potassium tertiary butoxide, sodium tertiary butoxide. The said reaction could be carried out in presence of inert solvent by using alkoxide as base. Preferably, N-methyl pyrrolidone is used as solvent and potassium tertiary butoxide is used as base. Potassium tertiary butoxide can be used in an amount of up to four molar equivalents for the said reaction. Preferably, two molar equivalents are used for conducting the said condensation reaction.

Reaction of thus obtained compound of formula (VI) with aqueous ammonia in refluxing dioxane gives the compound of formula (IVa). Surprisingly it was observed that because of absence of a bromo substituent on pyrimidine ring at 5-position, the said reaction goes to completion in 10 to 12 hrs instead of 96 hours as mentioned in the prior art process. Preferably, a 25% aqueous ammonia solution is used for the reaction. Though the reaction is carried out using dioxane as a solvent and a temperature of 120-130 °C, however, other inert solvents mentioned hereinbefore could be used for reaction.

Further, the present inventors have found that compound of formula (VI) can be purified by washing with ethyl acetate. Most of the undesired impurities, isomers are removed by the ethyl acetate solution washing. Typically, ethyl acetate washing treatment is done at 60-70°C followed by filtration at room temperature to get the desired product in pure form.

The obtained compound (IVa) then can be readily converted in the desired product etravirine by halogenating the same with free halogen e.g. free bromine or by using a halogen doner compounds. This halogenation reaction preferably is conducted in a suitable inert solvent. Preferred solvents are methylene dichloride or ether.

Thus, the present invention provides an efficient, simple and cost effective method for synthesis of etravirine. The process typically comprises the steps of:
1. Condensing 2,4,6-trichlorpyrimidine with 3,5-dimethyl-4-hydroxybenzonitrile, in presence of a base and inert solvent, to obtain compound of formula (V);
2. Converting the compound of formula (V) to a compound of formula (VI) by condensation with 4-aminobenzonitrile by using alkoxide as a base;
3. Optionally, purifying the compound of formula (VI);
4. Ammonlysis of compound of formula (VI) to get a compound of formula (IVa); and
5. Halogenation of compound of formula (IVa) in an inert solvent to get etravirine.

Thus, the present invention further provides a process for preparation of etravirine by using a compound of formula (V) and 4-aminobenzonitrile.

The present invention further provides a simple method for condensation of compound of formula (V) with 4-aminobenzonitrile.

The principles, preferred embodiments, and modes of operation of the present invention have been described in the foregoing specification. The invention which is intended to be protected herein, however, is not to be construed limited to the particular forms disclosed, since these are to be regarded as illustrative rather than restrictive. Variations and changes may be made by those skilled in the art, without departing from the spirit of the invention.

The invention is further explained with the help of following illustrative examples, however, in no way these examples should be construed as limiting the scope of the invention.

### EXAMPLES:

### Example 1

### Synthesis of 4-[(2,6-dichloro)-4-pyrimidinyloxy]-3,5 dimethylbenzonitrile (Compound-V):

2,4,6-Trichloropyrimidine (100 g, 0.545 m) was dissolved in 1,4-dioxane (300 ml) and 3,5,-dimethyl-4-hydroxybenzonitrile (80.1 g, 0.545m) was added under stirring. Addition of N,N-diisopropylethylamine (141.00g, 1.09m) was carried to this solution over a period of 30 minutes. Reaction mass was heated at 70°C and stirred for 2.0 hours. The reaction mass was cooled slowly to 15°C and obtained product was filtered at 12-15°C followed by washing the cake with 50 ml of 1,4-dioxane. Finally the cake was washed with water (200ml) to get the desired product. Melting point: 208-210°C.
Yield: 128 g, %Yield=80%;

### Example 2

### Synthesis of 4-[[6-chloro-2-[(4-cyanophenyl)amino]-4-pyrimidinyl]oxy]-3,5-dimethylbenzonitrile (Compound-VI)

Compound-V (100 g, 0.34 m) was dissolved in N-methylpyrolidone (500 ml) and 4-Aminobenzonitrile (40.12 g, 0.34m) was added under stirring. The reaction mass was cooled to 0°C. To this solution, addition of potassium t-butoxide was carried out (76.3g, 0.68m) in lots over a period of 1.0 hour at 0 to 10°C. The reaction mass was allowed to come at room temperature gradually over 1 to 2 hours. Then slowly the reaction mass was added in chilled water (2.0L) by maintaining the reaction mass temperature below 20°C. The reaction mass was filtered and washed the cake with 200 ml water. Wet cake was again dissolved in 1.0L water below 20°C and filtered. The obtained product was purified by using ethyl acetate (2x300 ml) at 60-70°C followed by filtration at 10-15°C.
Yield: 50 g.

### Example 3

### Synthesis of 4-[[6-amino-2-[(4-cyanophenyl)amino]-4-pyrimidinyl]oxy]-3,5-dimethylbenzonitrile (Compound - IVa)

Aqueous ammonia (25%) (600 ml) was added to a solution of Compound-VI (100 g, 0.266 m) in 1,4-Dioxane (1000 ml) and the reaction mass was heated in pressure autoclave at 120°C and maintain at 120-125°C for10-12 hours. The reaction mass was allowed to cool to 50°C, and again heated to 70-80°C, at which water (200 ml) was added slowly. The reaction mass gradually cooled to 10°C and filtered to obtain wet cake, which was dried to get desired product.
Yield: 75 g, %Yield= 80%.

### Example 4

### Synthesis of Etravirine

Compound-IVa (100 g, 0.28 m) was taken in methylene dichloride (800 ml) and cooled to a temperature of 0 to 5°C. Slowly liquid bromine (47.2 g, 0.294 m) was added at 0 to 5°C by dissolving in 200 ml of methylene dichloride. The reaction mass was stirred at 0 to 5°C for 2 to 4 hrs. Chilled water (800 ml) was added in to the reaction mass and pH was adjusted at 9 to 10 by slow addition of sodium hydroxide solution at 0 to 5°C. Sodium metabisulphite solution was added at 0 to 5°C and the reaction mass was stirred at 0-10°C for 1 hour by maintaining the reaction mass pH at 8 to 9. The reaction mass was filtered and washed the cake with 200 ml water. Dry the wet product at 50-60°C & recrystallize from acetone.
Yield: 100 g,
Melting point: 252 to 254°C.

## Claims

1. A method for synthesis of etravirine, comprising the steps of:
a. Condensing 2,4,6-trichlorpyrimidine with 3,5-dimethyl-4-hydroxybenzonitrile to obtain compound of formula (V); 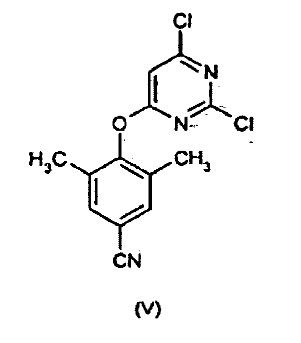
b. Converting the compound of formula (V) to a compound of formula (VI) by condensation with 4-aminobenzonitrile; 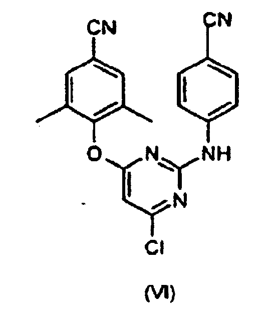
c. Optionally purifying the compound of formula (VI);
d. Ammonolysis of compound of formula (VI) to get a compound of formula (IVa); 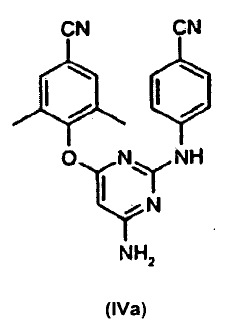 and
e. Halogenation of compound of formula (IVa) to get etravirine.

2. The method according to claim 1, wherein the step (a) is carried out in presence of an inert solvent and base.

3. The method according to claim 2, wherein the inert solvent is 1,4-dioxane and N,N-diisopropylethylamine is used as base.

4. The method according to claim 1, wherein the step (b) is carried out by using alkoxide as a base.

5. The method according to claim 4, wherein the base is potassium tertiary butoxide.

6. The method according to claim 1, wherein the step (b) is carried out using an inert solvent selected from ethanol, 1-methyl-2-pyrrolidone, N,N-dimethylformamide, 1,4-dioxane, tetrahydrofuran, dimethylsulfoxide, tetraline, sulfolane, and acetonitrile.

7. The method according to claim 6, wherein the inert solvent is 1-methyl-2-pyrrolidone.

8. The method according to claim 1, wherein the step (c) is carried out using ethyl acetate washing.

9. The method according to claim 1, wherein the step (d) is carried out using aqueous ammonia solution in 1,4-dioxane at a temperature of 120 -130 °C.

10. The method according to claim 1, wherein the step (e) is carried out using free halogen.

11. A compound being represented by formula V:

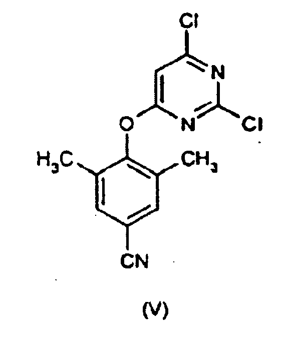

12. Use of a compound according to claim 11 as an intermediate in a method for synthesis of etravirine.

## Patentansprüche

1. Verfahren zur Synthese von Etravirin, umfassend die Schritte:
a. Kondensieren von 2,4,6-Trichlorpyrimidin mit 3,5-Dimethyl-4-hydroxybenzonitril, um eine Verbindung der Formel (V) zu erhalten; 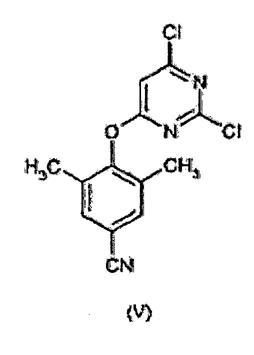
b. Umwandeln der Verbindung der Formel (V) in eine Verbindung der Formel (VI) durch Kondensation mit 4-Aminobenzonitril; 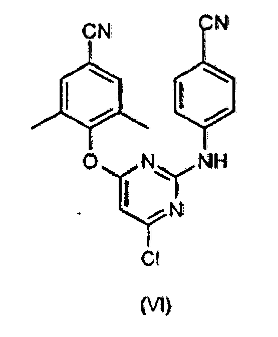
c. fakultativ Aufreinigen der Verbindung der Formel (VI);
d. Ammonolyse der Verbindung der Formel (VI), um eine Verbindung der Formel (IVa) zu erhalten; 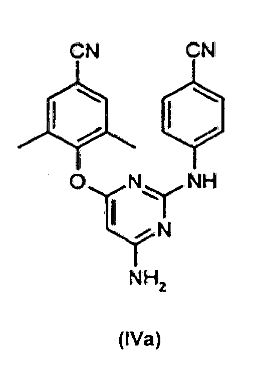 und
e. Halogenierung der Verbindung der Formel (IVa), um Etravirin zu erhalten.

2. Verfahren nach Anspruch 1, wobei der Schritt (a) durchgeführt wird in Anwesenheit eines inerten Lösungsmittels und einer Base.

3. Verfahren nach Anspruch 2, wobei das inerte Lösungsmittel 1,4-Dioxan ist und N,N-Diisopropylethylamin als Base verwendet wird.

4. Verfahren nach Anspruch 1, wobei der Schritt (b) durchgeführt wird unter Verwendung von Alkoxid als eine Base.

5. Verfahren nach Anspruch 4, wobei die Base Kalium-tert-Butoxid ist.

6. Verfahren nach Anspruch 1, wobei der Schritt (b) durchgeführt wird unter Verwendung eines inerten Lösungsmittels, ausgewählt aus Ethanol, 1-Methyl-2-pyrrolidon, N,N-Dimethylformamid, 1,4-Dioxan, Tetrahydrofuran, Dimethylsulfoxid, Tetralin, Sulfolan und Acetonitril.

7. Verfahren nach Anspruch 6, wobei das inerte Lösungsmittel 1-Methyl-2-pyrrolidon ist.

8. Verfahren nach Anspruch 1, wobei Schritt (c) durchgeführt wird unter Verwendung von Waschen mit Ethylacetat.

9. Verfahren nach Anspruch 1, wobei der Schritt (d) durchgeführt wird unter Verwendung einer wässrigen Ammoniaklösung in 1,4- Dioxan bei einer Temperatur von 120-130°C.

10. Verfahren nach Anspruch 1, wobei der Schritt (e) durchgeführt wird unter Verwendung von freiem Halogen.

11. Verbindung, dargestellt durch Formel V:

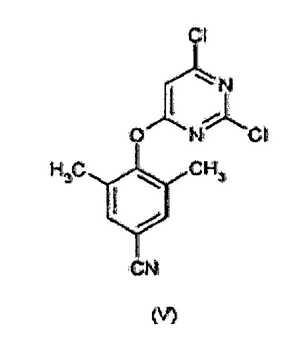

12. Verwendung einer Verbindung nach Anspruch 11 als ein Intermediat in einem Verfahren zur Synthese von Etravirin.

## Revendications

1. Procédé de synthèse d'étravirine, comprenant les étapes de :
a. condensation de 2,4,6-trichloropyrimidine avec du 3,5-diméthyl-4-hydroxybenzonitrile pour obtenir un composé de formule (V) ; 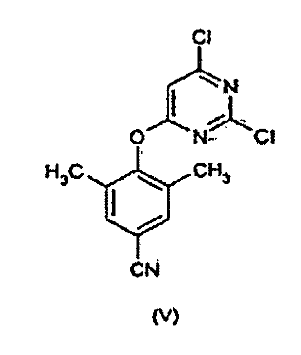
b. conversion du composé de formule (V) en un composé de formule (VI) par condensation avec du 4-aminobenzonitrile ; 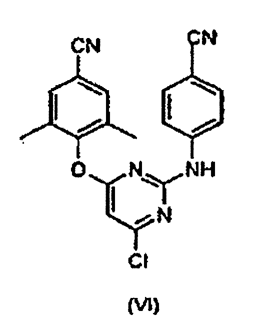
c. purification facultative du composé de formule (VI) ;
d. ammonolyse du composé de formule (VI) pour obtenir un composé de formule (IVa) ; 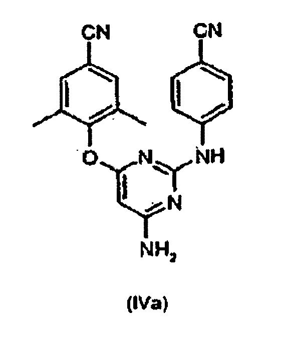 et
e. halogénation du composé de formule (IVa) pour obtenir l'étravirine.

2. Procédé selon la revendication 1, dans lequel l'étape (a) est réalisée en présence d'un solvant inerte et d'une base.

3. Procédé selon la revendication 2, dans lequel le solvant inerte est le 1,4-dioxane et la N,N-diisopropyléthylamine est utilisée comme base.

4. Procédé selon la revendication 1, dans lequel l'étape (b) est réalisée en utilisant un alcoxyde comme base.

5. Procédé selon la revendication 4, dans lequel la base est le butoxyde tertiaire de potassium.

6. Procédé selon la revendication 1, dans lequel l'étape (b) est réalisée en utilisant un solvant inerte choisi parmi l'éthanol, la 1-méthyl-2-pyrrolidone, le N,N-diméthylformamide, le 1,4-dioxane, le tétrahydrofurane, le diméthylsulfoxyde, la tétraline, le sulfolane et l'acétonitrile.

7. Procédé selon la revendication 6, dans lequel le solvant inerte est la 1-méthyl-2-pyrrolidone.

8. Procédé selon la revendication 1, dans lequel l'étape (c) est réalisée en utilisant un lavage à l'acétate d'éthyle.

9. Procédé selon la revendication 1, dans lequel l'étape (d) est réalisée en utilisant une solution d'ammoniaque aqueuse dans le 1,4-dioxane à une température de 120 à 130 °C.

10. Procédé selon la revendication 1, dans lequel l'étape (e) est réalisée en utilisant de l'halogène libre.

11. Composé représenté par la formule V :

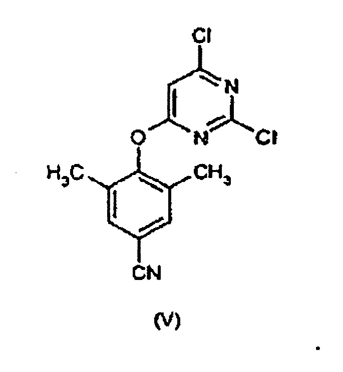

12. Utilisation d'un composé selon la revendication 11, comme intermédiaire dans un procédé de synthèse d'étravirine.
